# EUROPÄISCHE PATENTANMELDUNG

(11) **EP 3 372 263 A1**
(43) Veröffentlichungstag der Anmeldung: **12.09.2018**
(21) Anmeldenummer: 18153373.8
(22) Anmeldetag: 25.01.2018
(51) Int. Cl.: A61M 5/24, A61M 5/32

(54) **KANÜLE FÜR DIE INTRALIGAMENTÄRE ANÄSTHESIE**

(30) Priorität: 06.03.2017 DE 102017104618
(71) Anmelder: Henke-Sass, Wolf GmbH, 78532 Tuttlingen (DE)
(72) Erfinder: Müller, Fabian-Alexander, 78573 Wurmlingen (DE); Moser, Markus, 78054 Villingen-Schwenningen (DE); Taubenheim, Lothar, 40699 Erkrath (DE)
(74) Vertreter: Patentanwälte Geyer, Fehners & Partner mbB

(57) **Zusammenfassung**

Es wird eine Kanüle für die intraligamentäre Anästhesie bereitgestellt, wobei die Kanüle (1) einen rohrförmigen Körper (2) mit einem proximalen und einem distalen Ende (3, 4) aufweist, der Körper (2) eine proximale Öffnung (7) im proximalen Ende (3), eine distale Öffnung (8) im distalen Ende (4) und einen sich durch den Körper (2) erstreckenden Durchgang (6), der beide Öffnungen (7, 8) verbindet, umfasst,
wobei der Körper (2) einstückig ausgebildet ist und der Außendurchmesser (D1) des rohrförmigen Körpers (2) an keiner Stelle größer als 0,30 mm ist
und wobei sich das distale Ende (4) in Längsrichtung vom proximalen zum distalen Ende (3, 4) hin verjüngt sowie abgerundet ist und/oder frei von Schnittkanten ist.

## Beschreibung

Die vorliegende Erfindung betrifft eine Kanüle für die intraligamentäre Anästhesie sowie eine Spritze mit einer solchen Kanüle.

Bei der intraligamentären Anästhesie wird die Kanüle in den Peridontalspalt bzw. den Desmodontalspalt (Spalt zwischen Zahnhals und Zahnfleischsaum) so weit eingeführt, dass Knochenkontakt erreicht wird. Dann wird das Anästhetikum in den Peridontalspalt der entsprechenden Zahnwurzel injiziert, so dass der Zahnhalteapparat einschließlich der knöchernen Alveole bis zur Wurzelspitze des Zahnes mit dem Anästhetikum durchdrungen wird. Dadurch werden in wenigen Sekunden die in das Zahnmark eintretenden Nervenfasern betäubt. Bei der intraligamentären Anästhesie wird vorteilhaft wenig Anästhetikum pro Zahn verabreicht, was besonders bei Herz-Kreislauf-Risikopatienten von Vorteil sein kann. Da die Kanüle spitz angeschliffen ist, kann der Einstichschmerz relativ gering gehalten werden. Unabhängig davon tritt jedoch immer ein Einstichschmerz auf, da Gewebe verletzt wird, unabhängig davon, wie tief die Kanüle in den Desmodontalspalt eingeführt wird. Dies ist gerade bei Patienten mit Spritzenangst nachteilig, da jeder Zahn einzeln zu betäuben ist.

Ausgehend hiervon ist es daher Aufgabe der Erfindung, eine verbesserte Kanüle für die intraligamentäre Anästhesie bereitzustellen.

Die Erfindung ist in Anspruch 1 definiert. Vorteilhafte Ausgestaltungen sind in den abhängigen Ansprüchen angegeben.

Da bei der erfindungsgemäßen Kanüle sich das distale Ende in Längsrichtung vom proximalen zum distalen Ende hin verjüngt sowie abgerundet ist und/oder frei von Schnittkanten ist, ist es möglich, die Kanüle in den Peridontalspalt einzuführen, ohne Gewebe zu verletzen. Dieses Einführen kann so lange durchgeführt werden, bis Knochenkontakt erreicht wird. Somit kann das Verletzungsrisiko minimiert werden und es liegt kein Einstichschmerz vor.

Insbesondere kann das distale Ende kugelförmig abgerundet sein. Hierbei ist es bevorzugt, dass der Radius der kugelförmigen Abrundung die Hälfte des Außendurchmessers des rohrförmigen Körpers der Kanüle beträgt.

Das distale Ende kann so angeschliffen sein, dass es keinen Anschliffwinkel aufweist. Ferner kann das distale Ende so angeschliffen sein, dass es halbrund ist,

Der Außendurchmesser der Kanüle kann 0,30 mm oder kleiner sein. Bevorzugt sind Außendurchmesser von 0,300 mm sowie 0,28 mm. Der Innendurchmesse kann 0,133 mm, 0,165 mm oder auch 0,140 mm betragen. Damit liegen dann Wandstärken von 0,0835 mm, 0,0675 mm oder 0,08 mm beim Außendurchmesser von 0,3 mm und Wandstärken von 0,0735 mm, 0,0575 mm und 0,07 mm beim Außendurchmesser von 0,28 mm vor.

Der Durchgang erstreckt sich bevorzugt koaxial zur Längsachse.

Der rohrförmige Körper weist bevorzugt eine kreisringförmige Außenkontur im Querschnitt auf, so dass der Außendurchmesser eindeutig definiert ist. Bei einer anderen Außenkontur im Querschnitt wird unter Außendurchmesser der Durchmesser des kleinsten Kreisringes verstanden, in dem die Außenkontur des rohrförmigen Körpers vollständig enthalten ist.

Der rohrförmige Körper weist über seine gesamte Länge bevorzugt die gleiche Außenkontur im Querschnitt auf. Insbesondere ist der rohrförmige Körper als Hohlzylinder ausgebildet (jeweils bevorzugt mit Ausnahme des proximalen und des distalen Endes). Insbesondere weist der rohrförmige Körper einen konstanten Außendurchmesser über seine gesamte Länge auf.

Der rohrförmige Körper (mit dem distalen Ende oder gegebenenfalls ohne das proximale Ende) ist bevorzugt drehsymmetrisch zur Längsachse. Der rohrförmige Körper kann somit durch Drehung um bestimmte Winkel auf sich selbst abgebildet werden. Somit kann der rohrförmige Körper im Querschnitt grundsätzlich die Außenkontur einer dreieckigen, einer viereckigen oder einer sonstigen drehsymmetrischen polygonalen Fläche aufweisen. Bevorzugt ist jedoch die Außenkontur einer kreisförmigen Querschnittsform. Insbesondere kann der rohrförmige Körper rotationssymmetrisch zur Längsachse sein. In diesem Fall ist der Querschnitt kreisringförmig.

Der rohrförmige Körper weist somit bevorzugt zusammen mit seinem distalen Ende Drehsymmetrie oder Rotationssymmetrie (Abbildung auf sich selbst bei beliebigen Drehwinkeln um die Längsachse) auf. Das proximale Ende kann bevorzugt spitz und/oder so angeschliffen sein, dass es mindestens eine Schneidkante aufweist, so dass das proximale Ende nicht drehsymmetrisch ist.

Die Kanüle kann ferner einen Sockel aufweisen. Der Sockel kann z.B. dazu dienen, die Kanüle mit einer Spritze (z.B. einer Zylinderampullenspritze) zu verbinden. Der Sockel kann mit dem rohrförmigen Körper verklebt oder verschweißt sein. Ferner kann der Sockel drehsymmetrisch oder rotationssymmetrisch sein.

Das distale Ende kann so ausgebildet sein, dass der Radius der Abrundung in Richtung hin zum distalen Ende zunimmt.

Die Kanüle bzw. der rohrförmige Körper kann sich geradlinig erstrecken. Es ist ferner möglich, dass die Kanüle bzw. der rohrförmige Körper gebogen ist.

Es wird ferner eine Spritze mit einer erfindungsgemäßen Kanüle bereitgestellt. Die Spritze kann als Zylinderampullenspritze oder Karpulenspritze ausgebildet sein.

Es wird ferner ein Anästhesieverfahren beansprucht, bei dem eine Spritze mit einer erfindungsgemäßen Kanüle in den Peridontalspalt eingeschoben wird, wobei das distale Ende der Kanüle von der Spritze weg weist. Im eingeschobenen Zustand wird dann durch die Kanüle und über das distale Ende das Anästhetikum appliziert.

Das Verfahren kann weitere Schritte aufweisen, die im Zusammenhang mit der erfindungsgemäßen Kanüle und der Spritze mit der erfindungsgemäßen Kanüle beschrieben sind.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Nachfolgend wird die Erfindung beispielsweise anhand der beigefügten Zeichnungen, die auch erfindungswesentliche Merkmale offenbaren, noch näher erläutert. Es zeigen:
- Fig. 1: eine schematische Darstellung einer Ausführungsform der erfindungsgemäßen Kanüle 1:
- Fig. 2: eine vergrößerte Querschnittsansicht des distalen und proximalen Endes 4.3 des rohrförmigen Körpers 2 der Kanüle 1 von Fig. 1;
- Fig. 3: eine perspektivische Darstellung einer Spritze 11;
- Fig. 4: eine Schnittansicht der Spritze 11 von Fig. 3;
- Fig. 5: eine schematische Darstellung einer Zylinderampulle 23;
- Fig. 6: die Spritze 11 gemäß Fig. 3 und 4 mit der Zylinderampulle23 gemäß Fig. 5 und der Kanüle 1 gemäß Fig. 1 und 2;
- Fig. 7: eine schematische Darstellung eines Zahnes 40;
- Fig. 8: eine schematische Darstellung des Einführens der mit der Spritze 11 verbundenen Kanüle 1 in den Peridontalspalt 43;
- Fig. 9: eine Schnittansicht des distalen Endes 4 einer weiteren Ausführungsform der erfindungsgemäßen Kanüle, 1 und
- Fig. 10: eine Schnittansicht des distalen Endes 4 einer weiteren Ausführungsform der erfindungsgemäßen Kanüle 1.

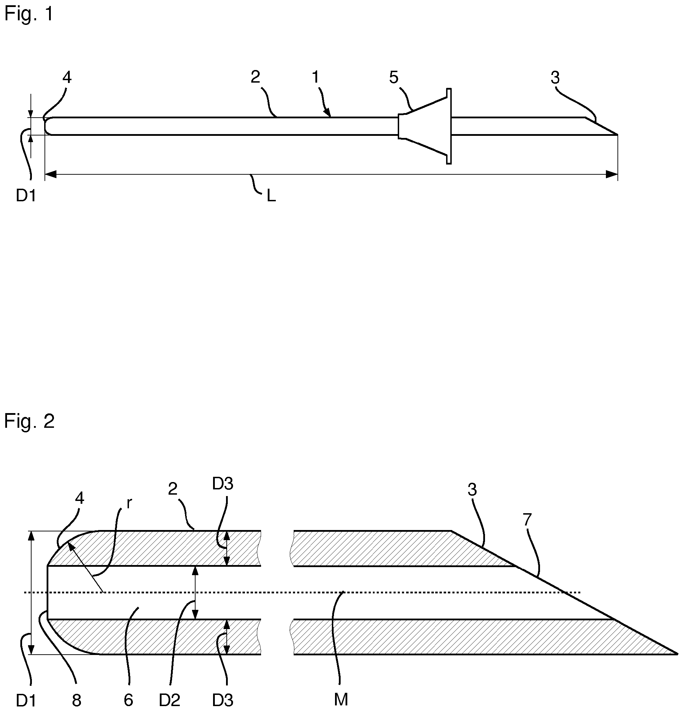

Bei der in Fig. 1 gezeigten Ausführungsform umfasst die erfindungsgemäße Kanüle 1 einen rohrförmigen Körper 2, der ein proximales Ende 3 und ein distales Ende 4 aufweist, und einen Sockel 5. Die erfindungsgemäße Kanüle 1 kann insbesondere bei der intraligamentären Anästhesie eingesetzt werden, bei der ein Anästhetikum über die Kanüle 1 appliziert wird, wobei die Kanüle 1 beispielsweise in dem Periodontalspalt an der Zahnwurzel des entsprechenden Zahnes eingeschoben sein kann.

Um dabei Gewebeverletzungen möglichst zu vermeiden, ist die erfindungsgemäße Kanüle 1 einerseits besonders dünn ausgebildet und ist andererseits das distale Ende 4 bei der beschriebenen Ausführungsform abgerundet. Des Weiteren ist der rohrförmige Körper 2 einstückig ausgebildet.

Wie insbesondere der Teilschnittansicht des proximalen und distalen Endes 3, 4 in Fig. 2 entnommen werden kann, weist der Körper 2 einen Durchgang 6 (der auch als Bohrung 6 bezeichnet werden kann) auf, der sich von einer proximalen Öffnung 7 am proximalen Ende des Körpers 2 bis zu einer distalen Öffnung 8 am distalen Ende 4 des Körpers 2 erstreckt. Der rohrförmige Körper 2 ist rotationssymmetrisch zu einer Mittelachse M, die sich vom proximalen bis zum distalen Ende 3, 4 erstreckt, und weist eine kreisförmige Außenkontur in einer Schnittebene senkrecht zur Zeichenebene in Fig. 2 auf. Ferner weist der Durchgang 6 in dieser Schnittebene einen kreisförmigen Querschnitt auf und erstreckt sich koaxial zur Mittelachse M.

Der rohrförmige Körper 2 weist einen Außendurchmesser D1 von kleiner oder gleich 0,30 mm auf. Bei der hier beschriebenen Ausführungsform beträgt der Außendurchmesser D1 0,30 mm. Der Innendurchmesser D2 des rohrförmigen Körpers 2 und somit der Durchmesser des Durchganges 6 beträgt 0,133 mm, so dass der rohrförmige Körper 2, der auch als Hohlnadel bezeichnet werden kann, eine Wandstärke D3 von 0,0835 mm aufweist.

Das proximale Ende 3 des Körpers 2 ist angeschliffen, wie in Fig. 2 dargestellt ist, um mit dem proximalen Ende 3 eine Membran einer Zylinderampulle durchstoßen zu können, wie nachfolgend noch beschrieben wird.

Das distale Ende 4 ist abgerundet. Bei der hier beschriebenen Ausführungsform ist das distale Ende kugelförmig angeschliffen, wobei der Radius r des Anschliffes bevorzugt die Hälfte des Außendurchmessers D1 und somit hier 0,15 mm beträgt.

Der rohrförmige Körper 2 kann aus Stahl oder Edelstahl hergestellt sein. Aufgrund der einstückigen Ausbildung und der durchgehenden Wandung liegt trotz des geringen Außendurchmessers die erforderliche Stabilität vor, selbst bei einer Länge L des Körpers 2 von z.B. 9 bis 15 mm. Der Körper 2 zeichnet sich insbesondere dadurch aus, dass er bis auf die beiden Öffnungen 7 und 8 keine weiteren Öffnungen aufweist. Es gibt somit insbesondere keine seitlichen Austrittsöffnungen in der Wandung, so dass der Körper 2 frei von seitlichen Austrittsöffnungen oder Eintrittsöffnungen ist. Insbesondere weist der rohrförmige Körper 2 einen konstanten Außendurchmesser über seine gesamte Länge auf, natürlich mit Ausnahme des abgerundeten distalen Endes 4, das sich verjüngt, und des proximalen Endes 3, das spitz angeschliffen ist. Ferner ist es bevorzugt, dass der Durchmesser des Durchgangs 6 über seine gesamte Länge konstant, so dass auch die Stärke der Wand des Körpers 2 konstant ist.

Der Sockel 5 kann aus Kunststoff oder Metall bestehen und ist mit dem rohrförmigen Körper 2 z.B. verklebt oder verschweißt.

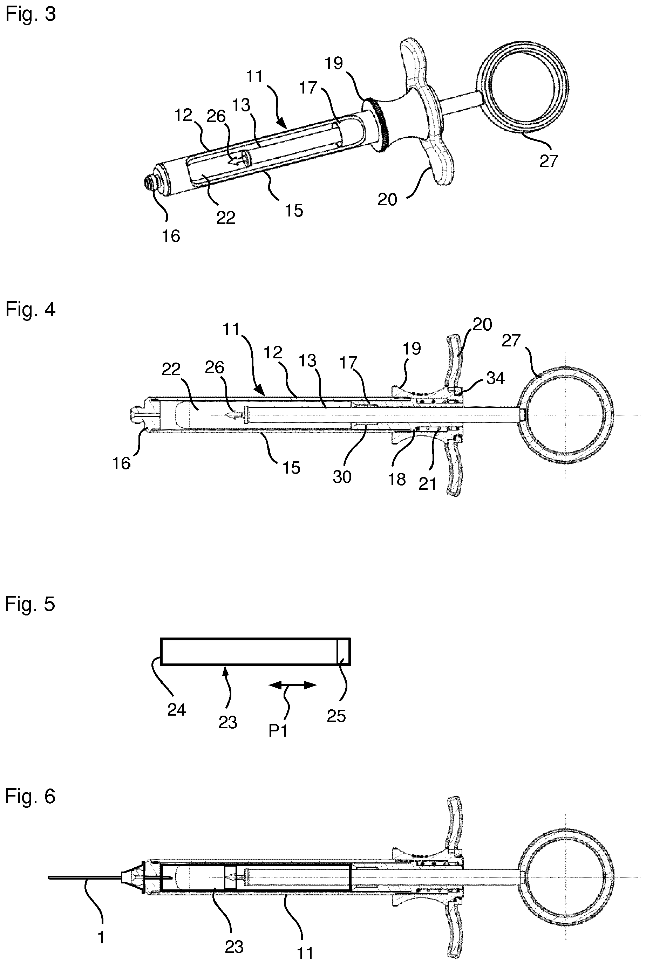

In Fig. 3 und 4 ist eine Spritze 11 gezeigt, die zusammen mit der erfindungsgemäßen Kanüle 1 verwendet werden kann. Die Spritze 11 kann z.B. als Zylinderampullenspritze, die auch als Karpulenspritze bezeichnet werden kann, ausgebildet sein. Die Spritze 11 umfasst einen im Wesentlichen rohrförmig ausgebildeten Spritzenkörper 12, in dem eine Kolbenstange 13 längsverschieblich gelagert ist.

Der Spritzenkörper 12 umfasst ein hohlzylinderförmiges Hauptrohr 15 mit einer Nadelaufnahme 16 am vorderen Ende und einer am hinteren Ende eingesetzten Zentrierhülse 17. Die Nadelaufnahme 16 ist in das Hauptrohr 15 eingeschraubt und verklebt. Die Zentrierhülse 17 ist bis zu ihrem Anschlag 18, der am hinteren Ende des Hauptrohrs 15 anliegt, in das Hauptrohr 15 eingeführt. Ferner ist am hinteren Ende des Hauptrohrs 15 eine Griffkappe 19 aufgeschraubt und verklebt, auf deren hinterem Ende eine Fingerstütze 20 drehbar gelagert und durch einen Ring 34 gesichert ist. Der Ring 34 ist dazu auf dem hinteren Ende der Fingerstütze 20 aufgeschraubt und verklebt. Zwischen der Zentrierhülse 17 und der Griffkappe 19 ist eine Spiralfeder 21 angeordnet, deren Enden mit der Zentrierhülse 17 und der Griffkappe 19 verbunden sind, so dass die Zentrierhülse 17 gegen das Hauptrohr 15 gedrückt wird.

Das Hauptrohr 15 weist eine seitliche Beladungsöffnung 22 auf, über die die Spritze 11 mit einer schematisch in Fig. 5 gezeigten Zylinderampulle 23 beladen werden kann. Bei der Zylinderampulle 23 kann es sich um eine herkömmliche Glasampulle handeln, die an ihrem vorderen Ende mit einer Membranschicht 24 und an ihrem hinteren Ende mit einer in der Längsrichtung der Zylinderampulle 23 verschiebbaren (wie durch den Doppelpfeil P1 angedeutet ist) Gummistopfen 25 verschlossen ist.

Die Kolbenstange 13 der Spritze 11 weist an ihrem vorderen Ende eine Pfeilspitze 26 auf, die in den Gummistopfen 25 der eingelegten Zylinderampulle 23 so eingestoßen werden kann, dass der Gummistopfen 25 durch die Bewegung der Kolbenstange 13 in Längsrichtung verschoben werden kann. Die Pfeilspitze 26 kann somit im Gummistopfen 25 verankert werden.

Am hinteren Ende der Kolbenstange 13 ist ein kreisringförmiger Ziehring 27 für den Daumen eines Benutzers der Spritze 11 befestigt. Die bisher beschriebenen Teile der Spritze 11 sind alle aus Metall hergestellt, wobei die Kolbenstange 13 aus Edelstahl besteht und die restlichen Teile (bis auf die Druckfeder 21) aus verchromtem Messing hergestellt sind. Der Spritzenkörper 12 weist eine Länge von ca. 110 mm auf und der Innendurchmesser des Hauptrohres beträgt ca. 10 mm. Die Kolbenstange 13 weist eine Länge von ca. 100 mm auf. Der Außen- und Innendurchmesser des Ziehrings 27 beträgt ca. 32 oder 30 mm.

Im Betrieb wird die Kolbenstange 13 mittels des Daumenabschnittes 27 so nach hinten gezogen, bis die Pfeilspitze 26 vollständig in einem Aufnahmebereich 30 am vorderen Ende der Zentrierhülse 17 versenkt ist. Ein weiteres Zurückziehen der Kolbenstange 13 führt dazu, dass die Zentrierhülse 17 in Längsrichtung des Hauptrohres 15 entgegen der Feder 21 verschoben wird, so dass ausreichend Platz im Hauptrohr 15 zum Einlegen der Zylinderampulle 23 vorliegt. In dieser Stellung wird die Kolbenstange 13 während des Einsetzens der Zylinderampulle 23 gehalten. Sobald die Zylinderampulle 23 über die seitliche Beladungsöffnung 22 in das Hauptrohr 15 eingesetzt wurde, kann das Halten der Stellung der Kolbenstange 13 beendet werden, so dass aufgrund der Rückstellkraft der Feder 21 die Zentrierhülse 17 die eingelegte Zylinderampulle 23 gegen die Nadelaufnahme 16 drückt und somit die Zylinderampulle 23 im Hauptrohr 15 fixiert.

Dann wird die Kolbenstange 13 nach vorne geschoben, um die Pfeilspitze 26 in den Gummistopfen 25 einzustechen. Ferner wird durch die Nadelaufnahme 16 und die Membran 24 die Kanüle 1 durch die Membranschicht 24 der Zylinderampulle 23 gestochen. Dies ist aufgrund des angeschliffenen bzw. angeschärften proximalen Endes 3 der Kanüle 1 leicht möglich. Dadurch ist die Spritze 11 für den Einsatz vorbereitet.

Bei der in Fig. 6 gezeigten Stellung ist der Gummistopfen 26 schon über die Hälfte bis zum vorderen Ende der Spritze 1 verschoben, so dass das entsprechende Fluid, das in der Zylinderampulle 23 vorhanden war, über die Kanüle 1 und somit über das distale Ende 4 abgegeben wurde. Bei dem Fluid kann es sich beispielsweise um ein Anästhetikum handeln.

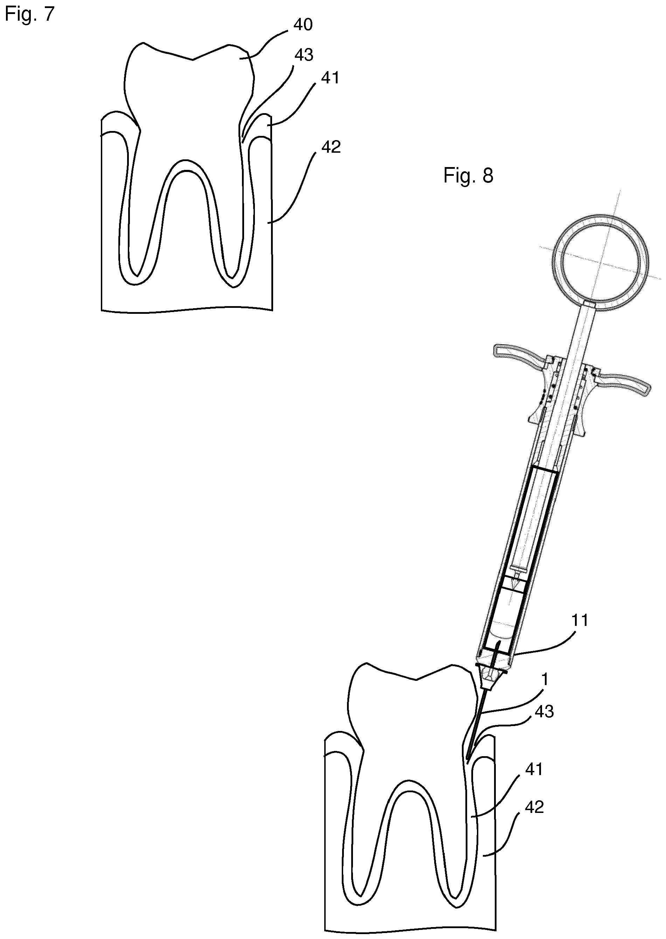

In Fig. 7 ist in einer schematischen Ansicht ein Zahn 40 zusammen mit dem Zahnfleisch 41 und dem entsprechenden Knochen 42 (z.B. Alveolarknochen) dargestellt. Zwischen dem Zahnfleisch 41 und dem Zahn 40 liegt der Periodontalspalt 43.

Um nun diesen Zahn 40 und den umgebenden Bereich des Zahnes 40 zu betäuben, wird die Spritze 11 so positioniert, dass das proximale Ende 3, das abgerundet ist, in den Peridontalspalt 43 eingeführt ist. Dies kann beispielsweise so erfolgen, dass Knochenkontakt erreicht ist. Damit ist die Kanüle 1 in die Bänder des Zahnhalteapparats, die Sharpey-Fasern, eingeführt. Dort kann dann das Anästhetikum über die Kanüle 1 injiziert werden. Das Anästhetikum kann den Zahnhalteapparat einschließlich der knöchernen Alveole bis zur Wurzelspitze des Zahnes durchdringen und betäubt dort in wenigen Sekunden die in das Zahnmark eintretenden Nervenfasern.

Da das distale Ende 4 abgerundet ist, kann die bisher immer auftretende Gewebeverletzung bei Kanülen mit angespitztem distalen Ende vermieden werden. Es können also Verletzungen vermieden werden und es tritt auch kein Einstichschmerz auf.

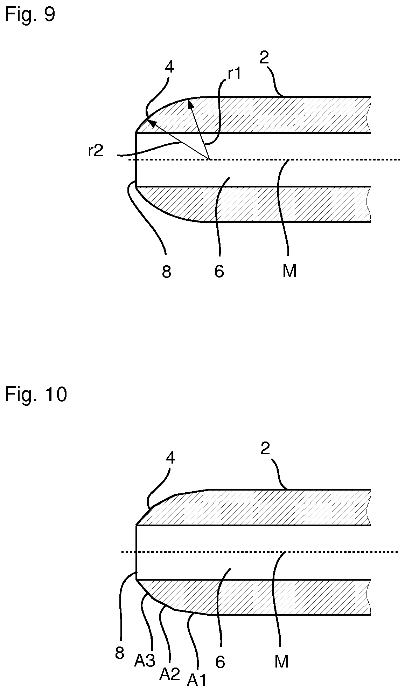

Das distale Ende 4 der Kanüle 1 muss nicht kugelförmig angeschliffen sein. Wesentlich ist, dass sich das distale Ende 4 in einer Richtung entlang der Mittelachse vom proximalen Ende 3 zum distalen Ende 4 hin verjüngt sowie frei von Schnittkanten und/oder abgerundet ist. Bei der in Fig. 9 gezeigten Ausführungsform nimmt der Krümmungsradius in dieser Richtung hin zu, so dass der Krümmungsradius r1 kleiner ist als der Krümmungsradius r2. Natürlich kann der Krümmungsradius in dieser Richtung auch abnehmen.

Des Weiteren ist es möglich, dass die abgerundete Form durch einen oder mehrere Abschnitte A1-A3 angenähert ist, wie in Fig. 10 schematisch dargestellt ist. Bei den Abschnitten A1-A3 kann es sich beispielsweise um Kegelmantelsegmente handeln.

## Patentansprüche

1. Kanüle für die intraligamentäre Anästhesie, wobei die Kanüle (1) einen rohrförmigen Körper (2) mit einem proximalen und einem distalen Ende (3, 4) aufweist,
der Körper (2) eine proximale Öffnung (7) im proximalen Ende (3), eine distale Öffnung (8) im distalen Ende (4) und einen sich durch den Körper (2) erstreckenden Durchgang (6), der beide Öffnungen (7, 8) verbindet, umfasst,
wobei der Körper (2) einstückig ausgebildet ist und der Außendurchmesser (D1) des rohrförmigen Körpers (2) an keiner Stelle größer als 0,30 mm ist
und wobei sich das distale Ende (4) in Längsrichtung vom proximalen zum distalen Ende (3, 4) hin verjüngt sowie abgerundet ist und/oder frei von Schnittkanten ist.

2. Kanüle nach Anspruch 1, bei der das distale Ende kugelförmig abgerundet ist.

3. Kanüle nach Anspruch 2, bei der das distale Ende (4) mit einem Radius abgerundet ist, der der Hälfte des Außendurchmessers des Körpers (2) beträgt.

4. Kanüle nach einem der obigen Ansprüche, bei dem das distale Ende mit einem Krümmungsradius abgerundet ist, der in Richtung hin zum distalen Ende (4) zunimmt.

5. Kanüle nach einem der obigen Ansprüche, wobei der Körper (2) drehsymmetrisch zur Längsachse oder rotationssymmetrisch zur Längsachse ist.

6. Kanüle nach einem der obigen Ansprüche, bei der das proximale Ende (3) angespitzt ist.

7. Kanüle nach einem der obigen Ansprüche, bei der der rohrförmige Körper (2) einen konstanten Außendurchmesser über seine gesamte Länge aufweist.

8. Kanüle nach einem der obigen Ansprüche, bei der der rohrförmige Körper neben der distalen und der proximalen Öffnung (7, 8) keine weiteren Öffnungen aufweist.

9. Kanüle nach einem der obigen Ansprüche, bei der der rohrförmige Körper aus Stahl besteht.

10. Spritze mit einer Kanüle nach einem der obigen Ansprüche.

11. Spritze nach Anspruch 10, wobei die Spritze als Zylinderampullenspritze ausgebildet ist.
